(19) [European Patent Office logo] Europäisches Patentamt / European Patent Office / Office européen des brevets

(11) **EP 1 845 803 B1**

(12) **EUROPEAN PATENT SPECIFICATION**

(45) Date of publication and mention
of the grant of the patent:
**30.04.2014 Bulletin 2014/18**

(21) Application number: **06710713.6**

(22) Date of filing: **20.01.2006**

(51) Int Cl.:
*A61K 8/11* *(2006.01)*　　*A61Q 13/00* *(2006.01)*
*A61Q 15/00* *(2006.01)*　　*A61Q 9/02* *(2006.01)*
*A23G 4/06* *(2006.01)*　　*A23L 1/00* *(2006.01)*
*A23L 1/035* *(2006.01)*　　*A23L 1/053* *(2006.01)*
*A61K 8/34* *(2006.01)*　　*A61K 8/73* *(2006.01)*
*C11D 1/66* *(2006.01)*　　*C11D 3/50* *(2006.01)*
*C11D 17/00* *(2006.01)*　　*A23L 1/22* *(2006.01)*
*C11D 11/02* *(2006.01)*

(86) International application number:
**PCT/IB2006/050225**

(87) International publication number:
**WO 2006/082536 (10.08.2006 Gazette 2006/32)**

(54) **METHOD FOR THE PREPRATION OF SPRAY-DRIED COMPOSITIONS AND THEIR USES**

VERFAHREN ZUR HERSTELLUNG VON SPRÜHGETROCKNETEN ZUSAMMENSETZUNGEN UND IHRE VERWENDUNGEN

PROCEDE DE PREPARATION DES COMPOSITIONS SECHEES PAR ATOMISATION ET UTILISATIONS

(84) Designated Contracting States:
**AT BE BG CH CY CZ DE DK EE ES FI FR GB GR HU IE IS IT LI LT LU LV MC NL PL PT RO SE SI SK TR**

(30) Priority: **03.02.2005 US 650181 P**

(43) Date of publication of application:
**24.10.2007 Bulletin 2007/43**

(73) Proprietor: **Firmenich SA
1211 Geneva 8 (CH)**

(72) Inventor: **SUBRAMANIAM, Anandaraman
East Windsor, New Jersey 08520 (US)**

(56) References cited:
**WO-A-03/043728　　US-A- 3 173 838
US-A- 3 971 852**

- **ARON S. MUJUMDAR: "Handbook of industrial drying", 1995**
- **Roberto Buffo & Gary Reineccius: "Comparison Among Assorted Drying Processes for the Encapsulation of Flavors" In: "Perfumer & Flavorist", 2001 vol. 26, pages 58-66,**

**Description**

**Technical Field**

The present invention relates to compositions in an encapsulated form and to a method for the preparation of said compositions.

**Background**

Encapsulation is a process by which one or more active ingredients are coated with, or entrapped within, another material or system. In the fragrance and flavor industries, the encapsulation of active ingredients serves to retain the aroma in a food or consumer product during storage, protects the active from undesirable interactions with the environments, particularly the food, minimizes perfume/perfume or flavor/flavor interactions, guards against either light induced reactions or oxidation, and provides a controlled release of the fragrance or flavor.

In this industry, the most common processes for producing encapsulated products are spray-drying and, to a lesser extent, extrusion and coacervation.

Spray drying is a well-known operation often employed by the flavor industry to render liquid flavors into a dry free-flowing powder form. It is a cost effective and efficient process to achieve this objective. The industry standard for the formulation consists of two major components: 80 parts of non-flavoring ingredient(s) and 20 parts of flavor, which is generally a hydrophobic liquid at room temperature. It is customary to add certain emulsifiers as part of the formulation if the non-flavoring ingredient lacks or is deficient in emulsifying properties.

In spray-dried powders, an active ingredient such as a flavor or a fragrance, usually hydrophobic, is entrapped as liquid droplets in a solidified matrix of a dehydrated carrier, generally consisting of carbohydrates, such as starches, hydrolyzed starches (maltodextrin), chemically modified starches, emulsifying polymers (gum Arabic) and in certain instances monomers and dimers of simple aldohexoses, or any combination thereof. Conventional spray-drying techniques are perfectly well documented in the prior art. See for example Spray-Drying Handbook, 4th ed., K. Masters, (1985) or other reference books on the subject-matter.

The method for the preparation of a spray-dried powder typically first comprises the dispersion of a carrier in water, and then the mixture of this dispersion with a fragrance or flavor before homogenization to form an oil-in-water emulsion. The emulsion is then spray-dried to produce a powdered product.

The present invention deals mainly with the preparation of spray-dried hydrophobic substances wherein the matrix consists essentially of gum Arabic and the contents of the finished powder product in hydrophobic encapsulate is above 25%, more preferably above 35% by weight, relative to the weight of finished product. Such spray-dried products are known as "high-fix" products, i.e. powders where the contents in hydrophobic material or encapsulated oil are above the 20-25% value that is conventional for this type of encapsulation technology.

Gum Arabic, also known as gum acacia, is a cost-effective, highly appreciated and widely used encapsulation material for spray-dried products. Its use is compatible with the regulatory requirements for Kosher and Halal certifications. Moreover, the fact that it does not have strong taste or aroma makes it a desirable carrier for the encapsulation of a large variety of flavor materials.

The gum consists of low molecular weight polysaccharides and a small amount of hydroxyproline-rich glycoprotein and minerals. This unique chemical composition leads to some of its unique functionalities which render it desirable for spray-drying. The relatively low molecular weight polysaccharides allow the preparation of gum solutions of low viscosity at high solids content. The glycoproteins provide the useful emulsifying properties of the gum. The latter also has an ability to form continuous films, is bland in taste and shows low hygroscopicity.

Although there is an abundance of literature describing the various factors affecting spray-drying efficiency upon the use of carbohydrate polymers as carriers, when it comes to the use of gum Arabic as carrier, by itself or in combination with other carbohydrates, there is much less information in the prior art.

In a recent study, B. F. McNamee et al., J. Agric. Food Chem. 49, 3385-3388, 2001, showed that partially replacing gum Arabic with maltodextrine could improve flavor retention by increasing the feeds solids supplied to the dryer. Y. Watanabe et al., J. Agric. Food Chem. 50, 3984-3987, 2002, described the effect of acyl ascorbate on the oxidation of linoleic acid in spray-dried system using maltodextrine and gum Arabic as carrier and showed that partial replacement of the latter by the former improved the oxidative stability of the powder. Finally, A. C. Bertolini et al., J. Agric. Food Chem. 49, 780-785, 2002, studied the oxidative degradation of monoterpenes in gum Arabic spray-dried products. All the commonly used monoterpenes tested, such as limonene, $\beta$-myrcene, $\beta$-pinene, citral and linalool, oxidized rapidly at 55 C, indicating the poor oxygen barrier properties of gum Arabic film.

It is also a known fact that there is volatile loss occurring during spray drying and that such losses increase with increasing fix levels. However, literature on high-fix spray drying is scarce.

In order to achieve high-fix levels of hydrophobic material, it is known to use special encapsulating matrices.

Formulations with high fix levels, typically above 50%, can be effectively spray dried with oil retentions in excess of 90% using modified starch (Octenyl Succinylated Starch - OSS) as a carrier. This has been described for instance in US 3,971,852. This patent relates primarily to carrier systems employing an emulsifying starch (OSS) in combination with low-molecular weight carbohydrates to achieve oxidative stability in the finished product. It also describes in an example the use of gum Arabic in combination with sorbitol and di-isooctyl sulfosuccinate (an emulsifier) to obtain a spray dried powder with 57.2% (w/w) oil content.

Nevertheless, the use of di-isooctyl sulfosuccinate (ionic surfactant/emulsifier) is not always possible for food applications for regulatory reasons.

Traditionally, when gum Arabic is used as the carrier, the aroma oil fix levels in such spray drying formulations is no more than 25% by weight, relative to the weight of spray-dried product. The reason for such a limitation is the excessive loss of the oil during the spray drying process when the fix level exceeds 25%.

For example, in a recent study carried out by the present inventors, for the encapsulation of menthol, mint oils or other mint flavor compositions in gum Arabic, the results indicated that, at or near conventional oil fix levels (20%), the retention of the menthol flavor on gum the Arabic carrier was only 89% at best.

The present invention aims at providing improved products spray-dried with gum Arabic as the carrier. The products of the invention carry an amount of encapsulated hydrophobic material above 25% by weight, and more preferably of at least 35% by weight, relative to the weight of the spray-dried product, with a retention level of the oil above 90% and, most times, close to a 100%.

## Summary of the Invention

The present invention now relates to a method for the preparation of a spray-dried powder according to claim 1. The composition comprises at least one active ingredient dispersed in a carrier of gum Arabic, wherein said active ingredient is present in an amount above 30% by weight, relative to the dried weight of the composition, the latter further comprising a non-ionic surfactant.

The composition further contains a fireproofing agent.

The active ingredient is menthol, a menthol-containing flavor or fragrance or a flavor or fragrance with a mint tonality. Such compositions are particularly advantageous in that they make it possible to impart to foods and other consumer products cooling and sensate properties, and to minimize irritation effects possibly associated with the organoleptic effect of menthol in particular.

The invention further relates to products comprising a spray-dried powder obtained by the method according to claim 1, and to its use for the flavoring or perfuming of consumer products such as foods, beverages, pharmaceutical or oral care compositions, or yet perfuming compositions.

The spray-dried powder prepared according to the invention can also be advantageously used as an intermediate product or starting product for a double-encapsulation method, i.e. as a solid product susceptible of being subjected to a further encapsulation such as an extrusion in a glassy matrix to provide a granular delivery system, or to a second spray-drying operation in a distinct or similar matrix, and the invention also relates to that use of the composition.

More objects, aspects and advantages of the invention will become apparent from the detailed description hereafter.

## Brief Description of the Drawings

Figure 1 represents the percentage of menthol retention in gum Arabic and maltodextrine based spray dried products as a function of the menthol concentration.

Figure 2 represents the amount of oil (menthol) that is solvent extractable (oil not encapsulated, remaining at the surface of the spray dried product), as a function of menthol concentration.

## Detailed Description of the Preferred Embodiments

The invention relates to a method for the preparation of a spray-dried powder, which comprises the steps of emulsifying the active ingredient in the gum Arabic and non-ionic emulsifier mixture; homogenizing the emulsion and spray drying the latter to form a particulate product.

The spray-dried composition comprises at least one active ingredient dispersed in a carrier of gum Arabic, wherein said active ingredient is present in an amount above 30% by weight, relative to the dried weight of the composition, the latter further comprising a non-ionic surfactant.

According to one embodiment of the invention, the emulsifier is preferably a food grade emulsifier, namely a di-acetyl tartaric acid ester of mono and diglycerides (DATEM). An example of the latter is the product commercialized under the tradename of Panodan by Danisco A/S, Langenbrogade 1, PO Box 17, Copenhagen, DK-1001.

As pointed out above, gum Arabic has very good emulsifying properties but limited emulsifying capacity. For this reason it works extremely well with respect to flavor retention when used as a carrier in spray drying. However, the performance of such a carrier can be adversely affected when an additional emulsifier/surfactant is added as part of the formulation. We have however found that the addition of a non-ionic surfactant has no detrimental effect on the spray drying of up to 50% by weight of mint flavours in gum Arabic matrices and this is a very surprising result.

The composition can be in the form of a spray-dried powder, comprising one or more active ingredients dispersed in a carrier, wherein said carrier consists essentially of a mixture of gum Arabic with a non-ionic surfactant.

The spray-dried product prepared according to the invention turned out to be very useful for encapsulating high amounts of hydrophobic liquid ingredients useful in the perfume and flavor industry, for obtaining powders of menthol and mint flavours or fragrances containing up 45% by weight of menthol, relative to the weight of the powder products. Such menthol, mint oils or other mint flavor composition powders are of very great importance for use in oral care products in particular, chewing gums and tablets for example, wherein the high concentration renders the use of the spray-dried mint flavor particularly cost-effective in application. They are also particularly useful for perfumed applications such as foaming shaving oils and antiperspirants.

The compositions prevent excessive oil loss during the spray-drying process, make it possible to obtain concentrations in active ingredients which are close to 50%, with a retention of nearly 100% during the common shelf-life periods, and this using carriers which fulfil wide regulatory criteria, namely for food products.

The composition contains at least 0.5% by weight of non-ionic emulsifier and between 30 and 50% by weight of active ingredient, relative to the weight of the composition.

Following an even more advantageous embodiment, the compositions will comprise from 2 to 10% by weight of emulsifier and at least 40% of active encapsulate, the remainder of the composition being gum Arabic.

Following a best embodiment of the invention there is provided a spray-dried composition consisting of 55 to 60% by weight of gum Arabic, 2 to 5% of DATEM and the remainder being menthol or a mint active composition, namely a mint flavor.

In the compositions there can be used any gum Arabic available commercially, under any one of its common names such as Turkey gum, Indian gum, Acacia Senegal, Gum #414 or gum Arabic. The nature and origin of the gum is not an essential parameter of the invention.

The essential characteristic of the invention thus resides in the combination of the gum Arabic with the non-ionic emulsifier, more particularly a DATEM. Other non-ionic emulsifiers that have proved appropriate according to the invention include Tween 80 (polyoxyethylesorbitan monooleate; commercialised by Uniquema, 3411 Silverside Road, Wilmington, DE 19803) and Glycosperse L20-K (polyoxyethylenesorbitan monolaurate; [commercialised by Lonza Inc., 90 Boroline Road, Allendale, NJ 07401]).

The compositions are in fact valuable for producing highly concentrated, free flowing powders, soluble in aqueous systems so as to release the active ingredient.

The active ingredient dispersed in the carrier of the spray-dried composition of the invention will preferably be a hydrophobic flavor or fragrance ingredient or composition of current use.

The terms "flavor and fragrance ingredient or composition" as used herein are selected from the group consisting of menthol, mint oil or a mint flavor. As previously mentioned, a preferred application relates to the encapsulation of mint oils.

One particular advantage of the process according to the invention lies in the fact that the compositions comprising a combination of gum Arabic with non-ionic emulsifier, as here-described, demonstrate a retention of the active ingredient (oil) which is advantageously not affected by a variation in the solid content of the starting aqueous emulsion. More particularly, in classical spray-drying processes, a high dilution rate, useful to provide mono-dispersity of the gum powder, must be balanced with the fact that the higher is the dilution rate, the lower will be the capability of the carrier film (after spray-drying) to retain the active oil, namely the flavor or fragrance. It is furthermore established that a solid content below 10% in the initial aqueous emulsion is expected to result in a noticeably decreasing active ingredient retention after spray-drying. Now, it turned out in the present case that, as shown in the examples below, the active hydrophobic encapsulate retention of the product of the invention is not affected by the dilution rate, or solid content in the aqueous emulsion introduced into the spray-drier. In other words, high dilution rates may be employed in order to get a good mono-dispersity of the carrier powder without provoking any drawback on the encapsulated oil retention, which is a totally unexpected result.

The spray-drying apparatus used in the process of the invention can be any one of the various commercially available apparatuses. Examples of spray-drying apparatuses are the Anhydro Dryers (origin: Anhydro Corp. of Attleboro Falls, Mass.), the Niro Dryer (manufactured by Niro Atomizer Ltd., Copenhagen, Denmark), or a Leaflash apparatus (origin : CCM Sulzer). Preferably a spray-drier with a pressure nozzle is used.

The process will be described in a more detailed manner in Example 1. However, the typical parameters of a spray-drying process are well known in the art and can be easily adjusted by a skilled person in the art.

The particles of the invention have typically a size comprised between 50 and 70 $\mu$m and a bulk density comprised between 0.4 and 0.6 g/cm$^3$. However, the granulometry and the bulk density of the resulting dry powders can be adjusted by selecting the nozzle (orifice size/diameter) and the atomization pressure so as to obtain the desired powder flowability.

The spray-dried powders prepared according to a process of the invention can be utilized as such in applications, for instance for the perfuming or flavoring of compositions wherein release is induced by contact with water or a source of humidity such as sweat, in perfumery applications, or saliva in the context of food intake or chewing tablets or gum. The products can be used cost effectively in any application where eventual water solubility of the carrier is desired resulting in the release of the active component.

One typical and very useful application is in the preparation of chewable tablets. In such tablet applications, upon the production of the tablets, a free-flowing flavor composition is critical when high-speed machines are employed. Yet, it is known that classical high-fix powders tend to be less free flowing than their low-fix counterparts primarily due to the un-encapsulated oil (solvent extractable oil) on the surface of the particle. The formulations result in low solvent extractable oil and thus yield a very free-flowing product with high flavor retention at high fix levels. This renders them particulary valuable when used in high-speed machines for portion packaging or tablet making.

The spray-dried compositions obtained according to the invention can also be advantageously used as starting materials to be subjected to further processing, before being used as delivery systems in a variety of applications.

In particular, in one embodiment of the invention, the spray-dried particles obtained by the method here-above described, can be further encapsulated in an extruded glassy matrix which may be formed from one or more carbohydrate materials or which may just comprise a plasticizer and an emulsifying agent, in order to produce a glassy matrix with an improved thermal stability. US 5,087,461, discloses an encapsulation method of a spray-dried composition by way of extrusion. This process leads to narrow rods having a diameter in the range of 0.3 to 3 mm.

Apart from the extrusion, other kinds of encapsulation processing, following the spray-drying, are possible. The powder obtained can for instance be submitted to a second spray-drying, thus providing a two-stage spray-dried product. This technique in particular, as well as multi-stage spray-drying in general, are described by W.J. Coumans, P.J.A.M. Kerkhof and S. Bruin in Drying Technology, Vol. 12 (1 and 2), (1994).

These examples are not restrictive of the encapsulation techniques which can be used for further processing of the spray-dried powder obtained according to the present invention, notably with the aim of increasing the size of the particles, or improving a particular characteristic of the powder, in order to rend it suitable for specific applications.

The compositions obtained according to the invention may also contain optional ingredients in addition to the gum Arabic carrier, the non-ionic emulsifier and the hydrophobic active ingredient. According to particularly useful embodiments, they further contain an effective amount of a fireproofing or explosion suppression agent susceptible of reducing the violence of powder explosion. The type and concentration of such agents in spray-drying compositions has been disclosed in detail in WO 03/043728 A1 (FIRMENICH). This document discloses perfuming or flavouring microcapsules having fireproofing agents dispersed in or absorbed within a polymeric carrier material, the fireproofing agents being basically inorganic salts. Reference is specifically made here to the teachings and examples in this document as they relate to the citation of the specific agents that can be used in the context of the present invention, the processes for their incorporation into the compositions of the invention and the typical concentrations for their use.

Other agents suitable as explosion suppressants, apart from those described in WO 03/043728, also possibly having further beneficial properties, are also contemplated according to the invention. They may for example be usable in smaller amounts, have a beneficial effect on the hygroscopicity of the microcapsules, or yet be particularly suitable for use in food applications.

For example, $C_1$-$C_{12}$ carboxylic acids, salts of $C_1$-$C_{12}$ carboxylic acids, and mixtures thereof can be directly added to perfuming and flavouring microcapsules in an amount effective to reduce the violence of possible explosions during their preparation, in particular when suspended in hot air, and during their storage.

The term "$C_1$-$C_{12}$ carboxylic acids" refers here to carboxylic acids containing 1 to 12 carbon atoms, including the C-atom of the carboxyl group. Therefore, if more than one carboxylic group is present in the carboxylic acid, the carbon of each carboxylic group shall be counted in the total number of carbon atoms mentioned.

The carboxylic acids and/or their salts possibly used as explosion suppressants in microcapsules of the present invention may be linear, branched, cyclic, and/or aromatic carboxylic acids and/or their salts. The carboxylic acids may be saturated or unsatured hydrocarbons.

Examples of cyclic carboxylic acids are lactones, for example ascorbic acid is a preferred such explosion suppression agent. An example of an appropriate aromatic carboxylic acid is salicylic acid

Preferably, the carboxylic acids and/or their salts are functionalised hydrocarbon carboxylic acids and/or their salts.

Preferably, the carboxylic acid comprise less than 7, more preferably less than 5 and most preferably less than 3 carbon atoms covalently bound to 2 hydrogen atoms.

Preferably, the carboxylic acid of the present invention comprises 5 or less carbon atoms with an oxidation number of (-II) or more negative. More preferably, the carboxylic acid comprises 3 or less carbon atoms having a oxidation number of (-II) or more negative.

In an embodiment of the present invention, the carboxylic acids and/or their salts are hydroxy- and/or ceto-functionalised hydrocarbon carboxylic acids and/or their salts.

Preferably, the carboxylic acid of the present invention comprises at least 1 hydroxy group. Preferably, it comprises at least 2 hydroxy groups.

In a further embodiment of the microcapsules of the present invention, the carboxylic acids and/or their salts are di-, tri-, or multi carboxylic acids and/or their salts. Examples of multi carboxylic acids include citric acid, which is $C_6$-tricarboxylic acid.

Preferably, the carboxylic acid is at least a di carboxylic acid More preferably, it is at least a tri carboxylic acid, meaning that it carries at least three carboxylic groups.

Preferably, the carboxylic acid and/or their salt is a $C_4$-$C_8$ carboxylic acid and/or their salt.

In a preferred embodiment of the present invention, the carboxylic acid and/or their salts are selected from $C_2$-$C_6$ carboxylic acids and/or their salts.

In preferred modes of carrying out the invention, the carboxylic acid and/or its salt is selected from the group consisting of acetic acid, propionic acid, butyric acid, isobutyric acid, valeric acid, caproic acid, citric acid, succinic acid, hydroxysuccinic acid, maleic acid, fumaric acid, oxylic acid, glyoxylic acid, adipic acid, lactic acid, tartaric acid, salicylic acid, ascorbic acid, the potassium, calcium and/or sodium salts of any of the afore-mentioned acids, and mixtures of any of these.

Preferably, the salts of the carboxylic acids are potassium and/or sodium salts. More preferably, there shall be used the tri-potassium and/or trisodium salt of a tricarboxylic acid. Most preferably, it is the tripotassium and/or trisodium salt of citric acid.

The above acids and their salts are commercially available. They can also be synthesized or extracted from natural materials by methods known per se.

Typically, the capsules will comprise from 2 to 15%, more preferably 2 to 10%, by weight of explosion suppressant, relative to the dry weight of the microcapsules.

The explosion suppressant can be added to the aqueous emulsion consisting of the perfuming or flavouring ingredient dispersed in the gum Arabic and non-ionic emulsifier. The obtained emulsion is then spray-dried in order to form the powder. This encapsulation technique does not require a more detailed description herein, as it relies on the conventional spray-drying techniques already mentioned earlier and which are perfectly well documented in the prior art [see for example Spray-Drying Handbook, 3rd ed., K. Masters ; John Wiley (1979)] and currently applied in the food industry or in the flavour and perfume industries.

In another embodiment, the explosion suppressant, in the form of a solid powder, is simply blended with the spray-dried powder prior obtained as described earlier from the aqueous emulsion of the hydrophobic aqueous ingredient or composition in the gum Arabic carrier and the non-ionic emulsifier.

The products prepared according to the invention, namely the spray-dried powder as such, as well as the spray-dried compositions further subjected to a second kind of encapsulation can be advantageously used for instance for the perfuming or flavoring of food compositions in particular.

Therefore, the delivery systems of the invention can be used in applications such as chewing gums or chewing sweets, chewing tablets, savory food or baking in the field of flavors. Similarly the field of perfumery comprises many applications where this kind of encapsulation systems may be very useful, particularly for detergents in tablet form, for foaming shaving oils and for antiperspirant sprays, powders and sticks.

The concentrations in which the microcapsules can be incorporated in the consumer products of the invention vary in a wide range of values, which are dependent on the nature of the product to be perfumed or flavoured. Typical concentrations, to be taken strictly by way of example, are comprised in a range of values as wide as from a few ppm up to 5 or 10% of the weight of the flavoring or perfuming composition or finished consumer product into which they are included.

The high-fix products prepared according to the invention, especially those having up to 50% weight content in active ingredient, prepared using gum Arabic in combination with a non-ionic surfactant/emulsifier, have substantially improved active ingredient, namely flavor, retention compared to their counterparts prepared using maltodextrins as carriers. They also yield powders with relatively low solvent extractable oil indicating efficient encapsulation especially at high active ingredient, in particular flavor, retention levels >95%). Some of the formulations and the analytical results are given below in the Examples.

The invention will now be described in a more detailed manner in the examples below, wherein the temperatures are indicated in degrees Celsius and the abbreviations have the usual meaning in the art.

## Examples

The following examples are further illustrative of the present invention embodiments, and further demonstrate the advantages of the invention devices relative to prior art teachings.

### Examples 1-9

Preparation of spray-dried compositions according to the invention and comparison with maltodextrine containing compositions

Nine powder formulations were prepared using the materials, equipment and general method conditions described hereafter.

**Materials:**

|        |                                                    |
|--------|----------------------------------------------------|
| Carrier: | Gum Arabic |
|        | Maltodextrin Morex 10-18 DE |
| Emulsifier: | Panodan - FDPK DATEM of soybean oil origin |
|        | Lamegin 39665 DWPS Cognis - DATEM of Sunflower oil origin |
|        | Tween 80K |
|        | Glycosperse L020K (Sorbitan Monococoate) |
| Flavor: | Menthol |

|        |                                                    |
|--------|----------------------------------------------------|
| **Equipment:** | Spray Dryer (box type) with nominal water evaporation rate of 60 kg/hr |
|        | Two-Stage homogenizer high-pressure pump |
|        | Clevenger Continuous Steam Distillation Apparatus |
|        | Gas Chromatograph - Hewlett Packard |

TABLE 1

| Sample # | % Ingredient | | | | | |
|---|---|---|---|---|---|---|
|  | Gum Arabic | Maltodextrin | Menthol | DATEM | Tween 80 | Glycosperse L20-K |
| **1** | 58.00 | - | 40.00 | 2.00 | - | - |
| **2** | 47.50 | - | 50.00 | 2.50 | - | - |
| **3** | 37.00 | - | 60.00 | 3.00 | - | - |
| **4\*** | - | 58.00 | 40.00 | 2.00 | - | - |
| **5\*** | - | 47.50 | 50.00 | 2.50 | - | - |
| **6\*** | - | 37.00 | 60.00 | 3.00 | - | - |
| **7** | 58.00 | - | 40.00 | - | 2.00 | - |
| **8** | 58.00 | - | 40.00 | - | - | 2.00 |
| **9** | 55.00 | - | 42.00 | 3.00 | - | - |
| * Comparison samples, using maltodextrine as the carrier | | | | | | |

**Spray Drying Method:**

The spray drying conditions were as given below:

Feed Solids = 40% (non aqueous matter)

Feed Temperature = 65°C
Homogenization Pressure = 100 bars
Feed Pressure = 170 - 200 bars
Dryer Inlet Temperature = 175°C
Dryer Outlet Temperature = 80°C
Dryer Air Flow Rate = 30 meter$^3$/minute
Spray Nozzle = 70/216 supplied by Spraying Systems Inc.

Samples were collected and analyzed as follows:

**Total Oil Determination:** Total oil was determined by continuous steam distillation, using the Clevenger apparatus, using a 10 g sample. One ml of toluene was added to the distillation flask to prevent crystallization of menthol in the side arm of the assembly. Distillation was done for two hours. Oil content was calculated by the following formula:

$$\textbf{Gravimetric oil content} = [(\text{ml Oil-1}) \times 0.89^{a}] / [\text{sample weight g}]$$

[a] Density of menthol

**Solvent Extractable Oil:** A GC-Internal Standard method described in literature by A. Scottitantawat et al, in J. Food Sci., Vol 68, No.7, 2003, p. 2256-2261, was modified and used. Extraction time was reduced to 10 seconds and 2-nonanone was used as an internal standard.

Table 2 summarizes the analytical results of the nine spray-dried menthol samples prepared by the method above with the respective materials indicated in Table 1.

TABLE 2

| Sample # | % Fix | % Oil content | % Retention | Extractable oil % |
|---|---|---|---|---|
| 1 | 40.00 | 40.05 | > 100.00 | 0.75 |
| 2 | 50.00 | 48.06 | 96.12 | 5.52 |
| 3 | 60.00 | 56.07 | 93.45 | 29.40 |
| 4* | 40.00 | 37.38 | 94.50 | 3.70 |
| 5* | 50.00 | 35.60 | 71.20 | 7.50 |
| 6* | 60.00 | 44.50 | 74.17 | 8.20 |
| 7 | 40.00 | 41.83 | > 100.00 | 0.08 |
| 8 | 40.00 | 39.60 | 99.00 | 0.04 |
| 9 | 42.00 | 40.50 | 96.43 | - |
| * Comparison samples, using maltodextrine as the carrier | | | | |

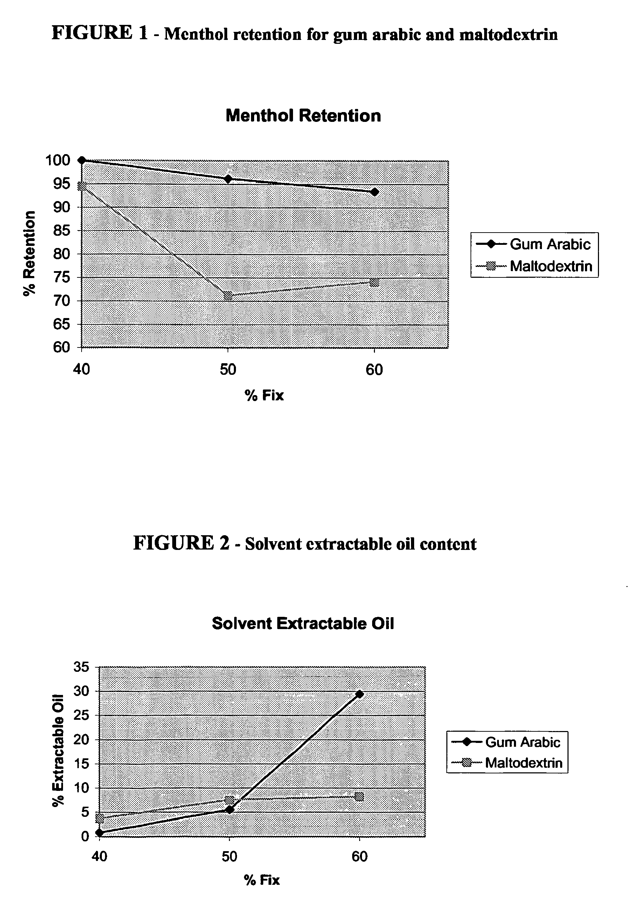

The results in this Table show clearly that the carrier system used in spray drying has a significant impact on menthol retention as well as the solvent extractable flavor/oil, gum Arabic showing consistent superiority, when compared to maltodextrin, both in its ability to to retain the oil, over a wide range of concentrations, and in the capability of reducing the level of solvent extractable oil for concentrations of encapsulated oil below 50% by weight. These effects are depicted graphically in Figures 1 and 2 respectively.

Solvent extractable oil is considered as un-encapsulated in dry powders. If the solvent extractable menthol was calculated as a percentage of total menthol in the powder it would suggest a very high (>98%) encapsulation. However, with increasing fix levels the amount of solvent extractable oil increases dramatically when the emulsifier is used at 5% level based on the menthol content and gum arabic is used as a carrier.

When using maltodextrin as carrier with the same emulsifier, the flavor loss is very pronounced when the fix level exceeds 40%, although the increase in solvent extractable oil is not very significant.

Example 10

Preparation of spray-dried compositions according to the invention, with different solids content

Proceeding as indicated in the preceding examples, there were prepared samples of encapsulated oils according to the invention, having the same dry weight composition as that of sample 1 in Table 2, by varying the feed solids weight percentage used in the process. The results of these tests are given here-below.

| Sample | % Solvent Extractable oil | Feed solids (% non-aqueous matter) | % Retention |
|---|---|---|---|
| A | 0.037 | 20 | 97.90 |
| B | 0.037 | 30 | 99.03 |
| C | 0.057 | 50 | 99.03 |

The above results show that varying the solid content of the starting emulsion, in the process of the invention, does not affect the retention of encapsulated oil.

Example 11

Preparation of a foaming shaving oil

A foaming shaving oil, perfumed via the use of gum Arabic capsules encapsulating a perfume, was prepared by admixing, in a generally known manner, the following ingredients.

| Ingredients | % by weight |
|---|---|
| Isopropyl palmitate | 49.00 |
| Trilaureth-4 phosphate | 2.00 |
| MIPA-laureth sulfate (and) laureth-4 (and) cocamide DEA | 42.30 |
| Sunflowerseed amidopropyl | 0.50 |
| Dimethylamine lactate bisabolol | 0.50 |
| Cocamide DEA | 3.00 |
| KATHON CG (Rohm & Haas) | 0.10 |
| Tocopherol (TENOX GT-2, Eastmann) | 0.50 |
| Dilauryl thiodipropionate | 0.10 |
| Perfume | 1.00 |
| Gum Arabic capsules | 1.00 |

Example 12

Preparation of an antiperspirant spray

An antiperspirant spray, perfumed via the use of gum Arabic capsules encapsulating a perfume, was prepared by admixing, in a generally known manner, the following ingredients.

| Ingredients | % by weight |
|---|---|
| Cyclomethicone | 11.75 |
| Isopropyl myristate | 2.20 |
| Silica | 0.25 |
| Quartenium-18 Hectorite | 0.80 |
| Aluminium chlorohydrate | 8.00 |
| Perfume | 0.40 |
| Gum Arabic capsules | 1.60 |
| Propane/Butane 2.5 bar | 75.00 |

Example 13

Preparation of an antiperspirant powder

An antiperspirant powder, perfumed with gum Arabic capsules encapsulating a perfume, was prepared by admixing, in a generally known manner, the following ingredients.

| Ingredients | % by weight |
| --- | --- |
| Talcum | 87.00 |
| Aluminium chlorohydrate | 10.00 |
| Perfume | 1.50 |
| Gum Arabic capsules | 1.50 |

Example 14

Preparation of an antiperspirant stick

An antiperspirant stick, perfumed via the use of gum Arabic capsules encapsulating a perfume, was prepared by admixing, in a generally known manner, the following ingredients.

| Ingredients | % by weight |
| --- | --- |
| Cyclomethicone | 48.50 |
| Steraryl alcohol | 21.00 |
| PPG-14 Butyl ether | 2.00 |
| Hydrogenated Caster oil | 1.00 |
| Octyldodecanol | 4.00 |
| Aluminium chlorohydrate | 21.00 |
| Perfume | 1.00 |
| Gum Arabic capsules | 1.50 |

Example 15

Preparation of a chewing gum

A chewing gum, flavored via the use of gum Arabic capsules encapsulating a flavor, was prepared by admixing, in a generally known manner, the following ingredients.

| Ingredients | % by weight |
| --- | --- |
| Confectioners sugar | 59.35 |
| Gum Base-Nova Base T | 19.40 |
| Corn syrup, 42 DE | 19.80 |
| Citric acid | 0.70 |
| Glycerine | 0.50 |
| Gum Arabic capsules | 0.25 |

**Claims**

1. A method for the preparation of a spray-dried powder, comprising the steps of emulsifying at least one flavor or fragrance material in a mixture of gum Arabic and a non-ionic emulsifier, homogenizing the emulsion and spray drying the homogenized emulsion to form a particulate product, wherein the flavor or fragrance material is present in an amount above 30% by weight, relative to the dried weight of the product_and wherein said flavor or fragrance material is selected from the group consisting of menthol, mint oil or a mint flavor.

**2.** A method according to claim 1, wherein said non-ionic surfactant is a di-acetyl tartaric acid ester of mono- or di-glycerides.

**3.** A method according to claim 1, comprising 2 to 15% by weight of a non-ionic surfactant selected from the group consisting of the di-acetyl tartaric acid esters of mono- and di-glycerides, polyoxyethylenesorbitan monooleate and polyoxyethylenesorbitan monolaurate type surfactants, relative to the dried weight of the composition.

**4.** A method according to claim 1, which comprises from 30 to 50% by weight of said flavor or fragrance, relative to the dried weight of the composition.

**5.** A method according to claim 4 wherein said non-ionic surfactant is a di-acetyl tartaric acid ester of mono- or di-glycerides.

**6.** A method according to claim 5, wherein said non-ionic surfactant is present in a concentration comprised between 2 and 10% by weight, relative to the dried weight of the composition.

**7.** A method according to claim 1, which further comprises an explosion suppression agent.

**8.** A method according to claim 7, wherein said explosion suppression agent is selected from the group consisting of sodium silicate, potassium silicate, sodium carbonate, sodium hydrogencarbonate, monoammonium phosphate or carbonate, diammonium phosphate, mono-, di- or trisodium phosphate, sodium hypophosphite, melamine cyanurate, chlorinated hydrocarbons and mixtures thereof.

**9.** A method according to claim 7, wherein said explosion suppression agent is selected from the group consisting of acetic acid, propionic acid, butyric acid, isobutyric acid, valeric acid, caproic acid, citric acid, succinic acid, hydroxysuccinic acid, maleic acid, fumaric acid, oxylic acid, glyoxylic acid, adipic acid, lactic acid, lactic acid, tartric acid, salicylic acid, ascorbic acid, the potassium, calcium and/or sodium salts of any of the afore-mentioned acids, and mixtures of any of these.

**10.** A method according to claim 7, comprising from 5 to 15% by weight of said explosion suppression agent, relative to the dried weight of the composition.

**11.** A product selected from the group consisting of a food, a beverage, a nutrition supplement, an oral care composition, a deodorant, a soap, a detergent tablet and a chewing tablet or gum, which product comprises a spray-dried powder prepared according to claim 1 in an amount sufficient to impart, improve, modify or enhance the taste, odor and/or nutritional value thereof.

**12.** A method for flavoring, perfuming and/or fortifying a product selected from the group consisting of a food, a beverage, a nutrition supplement, an oral care composition, a tablet or a chewing gum, a soap or detergent tablet, a body deodorant or foam shaving oil, which method comprises adding a spray-dried powder prepared according to claim 1 thereto, in an amount sufficient to impart, improve, modify or enhance the taste, odor and/or nutritional value of said product.

**Patentansprüche**

**1.** Verfahren für die Herstellung eines sprühgetrockneten Pulvers, umfassend die Schritte, mindestens ein Aroma- oder Duftmaterial in einem Gemisch von Gummi arabicum und einem nicht-ionischen Emulgator zu emulgieren, die Emulsion zu homogenisieren und die homogenisierte Emulsion sprühzutrocknen, um ein teilchenförmiges Produkt zu bilden, wobei das Aroma- oder Duftmaterial in einem Anteil über 30 Gew.-%, relativ zu dem Trockengewicht des Produkts, vorhanden ist und wobei das Aroma- oder Duftmaterial aus der Gruppe, bestehend aus Menthol, Minzöl oder einem Minzaroma, ausgewählt ist.

**2.** Verfahren gemäß Anspruch 1, wobei das nicht-ionische grenzflächenaktive Mittel ein Diacetylweinsäureester von Mono- oder Diglyceriden ist.

**3.** Verfahren gemäß Anspruch 1, umfassend 2 bis 15 Gew.-% von einem nicht-ionischen grenzflächenaktiven Mittel, ausgewählt aus der Gruppe, bestehend aus den Diacetylweinsäureestern von Mono- und Diglyceriden, grenzflächenaktiven Mitteln vom Polyoxyethylensorbitanmonooleat- und Polyoxyethylensorbitanmonolaurat-Typ, relativ zu dem Trockengewicht der Zusammensetzung.

4. Verfahren gemäß Anspruch 1, welches von 30 bis 50 Gew.-% von dem Aromastoff oder Duftstoff, relativ zu dem Trockengewicht der Zusammensetzung, umfasst.

5. Verfahren gemäß Anspruch 4, wobei das nicht-ionische grenzflächenaktive Mittel ein Diacetylweinsäureester von Mono- oder Diglyceriden ist.

6. Verfahren gemäß Anspruch 5, wobei das nicht-ionische grenzflächenaktive Mittel in einer Konzentration zwischen 2 und 10 Gew.-%, relativ zu dem Trockengewicht der Zusammensetzung, vorhanden ist.

7. Verfahren gemäß Anspruch 1, welches weiterhin ein Explosionsunterdrückungsmittel umfasst.

8. Verfahren gemäß Anspruch 7, wobei das Explosionsunterdrückungsmittel aus der Gruppe, bestehend aus Natriumsilicat, Kaliumsilicat, Natriumcarbonat, Natriumhydrogencarbonat, Monoammoniumphosphat oder -carbonat, Diammoniumphosphat, Mono-, Di- oder Trinatriumphosphat, Natriumhypophosphit, Melamincyanurat, chlorierten Kohlenwasserstoffen und Gemischen davon, ausgewählt ist.

9. Verfahren gemäß Anspruch 7, wobei das Explosionsunterdrückungsmittel aus der Gruppe, bestehend aus Essigsäure, Propionsäure, Buttersäure, Isobuttersäure, Valeriansäure, Capronsäure, Citronensäure, Bernsteinsäure, Hydroxybernsteinsäure, Maleinsäure, Fumarsäure, Oxalsäure, Glyoxalsäure, Adipinsäure, Milchsäure, Weinsäure, Salicylsäure, Ascorbinsäure, den Kalium-, Calcium- und/oder Natriumsalzen von einer der vorstehend erwähnten Säuren und Gemischen von allen von diesen, ausgewählt ist.

10. Verfahren gemäß Anspruch 7, umfassend von 5 bis 15 Gew.-% von dem Explosionsunterdrückungsmittel relativ zu dem Trockengewicht der Zusammensetzung.

11. Produkt, ausgewählt aus der Gruppe, bestehend aus einem Nahrungsmittel, einem Getränk, einem Nahrungsergänzungsmittel, einer Mundpflegezusammensetzung, einem Deodorant, einer Seife, einer Detergenstablette und einer Kautablette oder einem Kaugummi, welches Produkt ein sprühgetrocknetes Pulver, hergestellt gemäß Anspruch 1, in einer Menge, ausreichend, um den Geschmack, Geruch und/oder Nährwert davon zu vermitteln, zu verbessern, zu modifizieren oder zu erhöhen, umfasst.

12. Verfahren zum Aromatisieren, Parfümieren und/oder Anreichern eines Produkts, ausgewählt aus der Gruppe, bestehend aus einem Nahrungsmittel, einem Getränk, einem Nahrungsergänzungsmittel, einer Mundpflegezusammensetzung, einer Tablette oder einem Kaugummi, einer Seife oder Detergenstablette, einem Körperdeodorant oder Schaumrasieröl, welches Verfahren umfasst, ein sprühgetrocknetes Pulver, hergestellt gemäß Anspruch 1, in einer Menge, ausreichend, um den Geschmack, Geruch und/oder Nährwert des Produkts zu vermitteln, zu verbessern, zu modifizieren oder zu erhöhen, dazu hinzuzufügen.


**Revendications**

1. Procédé de préparation d'une poudre séchée par atomisation, comprenant les étapes consistant à émulsionner au moins une substance d'arôme ou de parfum dans un mélange de gomme arabique et d'un émulsifiant non ionique, homogénéiser l'émulsion et sécher par atomisation l'émulsion homogénéisée pour former un produit particulaire, dans lequel la substance d'arôme ou de parfum est présente en une quantité de plus de 30 % en poids par rapport au poids à sec du produit et dans lequel la substance d'arôme ou de parfum est sélectionnée parmi le groupe consistant en menthol, huile de menthe ou un arôme de menthe.

2. Procédé selon la revendication 1, dans lequel ledit tensioactif non ionique est un ester diacétylique d'acide tartrique de monoglycérides ou de diglycérides.

3. Procédé selon la revendication 1, comprenant 2 à 15 % en poids d'un tensioactif non ionique sélectionné parmi le groupe consistant en les tensioactifs du type esters diacétyliques d'acide tartrique de monoglycérides et de diglycérides, polyoxyéthylènesorbitan monooléate et polyoxyéthylènesorbitan monolaurate, par rapport au poids à sec de la composition.

**4.** Procédé selon la revendication 1, qui comprend de 30 à 50 % en poids dudit arôme ou parfum par rapport au poids à sec de la composition.

**5.** Procédé selon la revendication 4, dans lequel ledit tensioactif non ionique est un ester diacétylique d'acide tartrique de monoglycérides ou de diglycérides.

**6.** Procédé selon la revendication 5, dans lequel ledit tensioactif non ionique est présent en une concentration comprise entre 2 et 10 % en poids par rapport au poids à sec de la composition.

**7.** Procédé selon la revendication 1, qui comprend en outre un agent de suppression d'explosion.

**8.** Procédé selon la revendication 7, dans lequel ledit agent de suppression d'explosion est sélectionné parmi le groupe consistant en silicate de sodium, silicate de potassium, carbonate de sodium, hydrogénocarbonate de sodium, phosphate ou carbonate de monoammonium, phosphate de diammonium, phosphate monosodique, disodique ou trisodique, hypophosphite de sodium, cyanurate de mélamine, hydrocarbures chlorés et des mélanges de ceux-ci.

**9.** Procédé selon la revendication 7, dans lequel ledit agent de suppression d'explosion est sélectionné parmi le groupe consistant en acide acétique, acide propionique, acide butyrique, acide isobutyrique, acide valérique, acide caproïque, acide citrique, acide succinique, acide hydroxysuccinique, acide maléique, acide fumarique, acide oxylique, acide glyoxylique, acide adipique, acide lactique, acide tartrique, acide salicylique, acide ascorbique, les sels de potassium, de calcium et/ou de sodium de l'un quelconque des acides susmentionnés et des mélanges de quelconques de ceux-ci.

**10.** Procédé selon la revendication 7, comprenant de 5 à 15 % en poids dudit agent de suppression d'explosion par rapport au poids à sec de la composition.

**11.** Produit sélectionné parmi le groupe consistant en un aliment, une boisson, un supplément nutritif, une composition de soin oral, un déodorant, un savon, une pastille détergente et une pastille ou une gomme à mâcher, lequel produit comprend une poudre séchée par atomisation préparée selon la revendication 1, en une quantité suffisante pour conférer, améliorer, modifier ou rehausser le goût, l'odeur et/ou la valeur nutritive de celui-ci.

**12.** Procédé destiné à aromatiser, parfumer et/ou fortifier un produit sélectionné parmi le groupe consistant en un aliment, une boisson, un supplément nutritif, une composition de soin oral, une pastille ou un chewing-gum, un savon ou une pastille détergente, un déodorant corporel ou une huile pour mousse à raser, lequel procédé comprend ajouter une poudre séchée par atomisation préparée selon la revendication 1 à celui-ci en une quantité suffisante pour conférer, améliorer, modifier ou rehausser le goût, l'odeur et/ou la valeur nutritive dudit produit.

## FIGURE 1 - Menthol retention for gum arabic and maltodextrin

**Menthol Retention**

## FIGURE 2 - Solvent extractable oil content

**Solvent Extractable Oil**

## REFERENCES CITED IN THE DESCRIPTION

**Patent documents cited in the description**

- US 3971852 A
- US 5087461 A
- WO 03043728 A1, FIRMENICH
- WO 03043728 A

**Non-patent literature cited in the description**

- **K. MASTERS.** Spray-Drying Handbook. 1985
- **B. F. MCNAMEE et al.** *J. Agric. Food Chem.,* 2001, vol. 49, 3385-3388
- **Y. WATANABE et al.** *J. Agric. Food Chem.,* 2002, vol. 50, 3984-3987
- **A. C. BERTOLINI et al.** *J. Agric. Food Chem.,* 2002, vol. 49, 780-785
- **W.J. COUMANS, P.J.A.M. KERKHOF ; S. BRUIN.** *Drying Technology,* 1994, vol. 12 (1, 2
- **K. MASTERS.** Spray-Drying Handbook. John Wiley, 1979
- **A. SCOTTITANTAWAT et al.** *J. Food Sci.,* 2003, vol. 68 (7), 2256-2261